(19) Europäisches Patentamt
European Patent Office
Office européen des brevets

(11) Numéro de publication: **0 254 611 B1**

(12) # FASCICULE DE BREVET EUROPEEN

(45) Date de publication de fascicule du brevet: **12.06.91**

(51) Int. Cl.$^5$: **C07D 215/36**, A61K 31/47

(21) Numéro de dépôt: **87401466.5**

(22) Date de dépôt: **25.06.87**

(54) **Dérivés 8-phénylthiotétrahydroquinoléines substitués et leurs sels, leur préparation à titre de médicaments et les compositions les renfermant.**

(30) Priorité: **25.06.86 GB 8615563**

(43) Date de publication de la demande:
**27.01.88 Bulletin 88/04**

(45) Mention de la délivrance du brevet:
**12.06.91 Bulletin 91/24**

(84) Etats contractants désignés:
**AT BE CH DE ES FR GR IT LI LU NL SE**

(56) Documents cités:
**GB-A- 2 069 493**

**JOURNAL OF MEDICINAL CHEMISTRY, vol. 26, no. 2, février 1983, pages 218-222, American Chemical Society, US; F. HAVIV et al.: "2-[(Phenylthio)methyl]pyridine derivatives: new antiinflammatory agents"**

(73) Titulaire: **ROUSSEL-UCLAF**
**35, boulevard des Invalides**
**F-75007 Paris(FR)**

(72) Inventeur: **Clements-Jewery, Stephen**
**14 Church Walk**
**Ashton Keynes Wiltshire(GB)**
Inventeur: **Kennewell, Peter David**
**10 St. Helen's View Okus**
**Swindon Wiltshire(GB)**
Inventeur: **Westwood, Robert**
**Martagon 6 Rimes Close**
**Kingston Bagpuize Oxfordsire(GB)**

(74) Mandataire: **Fritel, Hubert et al**
**111, route de Noisy B.P. no 9**
**F-93230 Romainville(FR)**

**EP 0 254 611 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouvelles 8-phénylthiotétrahydroquinoléines substituées et leurs sels, ainsi que le procédé de préparation et l'application à titre de médicament de ces nouveaux produits.

Le document 3 de F. Haviv et Coll. (Journal of Medicinal Chemistry, Vol. 26 n° 2 (1983) pages 218 à 222) décrit des dérivés de la 2-[(phénylthio) méthyl] pyridine ayant des propriétés anti-inflammatoires.

L'invention a pour objet de nouvelles 8-phénylthiotétrahydroquinoléines substituées répondant à la formule (I) :

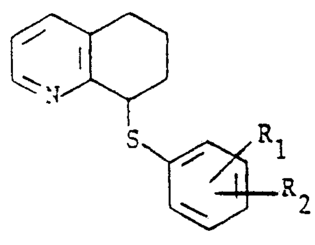

(I)

dans laquelle $R_1$ et $R_2$, identiques ou différents, représentent soit un atome d'hydrogène, soit un atome d'halogène, soit un radical alcoyle renfermant de 1 à 6 atomes de carbone éventuellement substitué par un radical hydroxyle, soit un radical alcoxyle renfermant de 1 à 6 atomes de carbone, soit un radical alcanoyloxy renfermant de 1 à 6 atomes de carbone, soit un radical alcoxycarbonyl renfermant de 2 à 6 atomes de carbone, soit un radical hydroxy, nitro, amino, carboxy, cyano ou amino-sulfonyl, soit lorsque $R_1$ et $R_2$ sont portés par des atomes de carbone adjacents, forment avec ceux-ci un radical phényle, ainsi que leurs sels d'addition avec les acides.

Dans la formule générale (I) et dans ce qui suit, le terme halogène désigne notamment un atome de chlore ou de brome, le terme radical alcoyle renfermant de 1 à 6 atomes de carbone désigne, par exemple, un radical méthyle, éthyle, propyle, isopropyle, butyle linéaire ou ramifié, pentyle linéaire ou ramifié, hexyle linéaire ou ramifié ;

- le terme radical alcoyle substitué par un radical hydroxy désigne par exemple un radical hydroxyméthyle, 2-hydroxyéthyle, 3-hydroxypropyle, 4-hydroxybutyle, 5-hydroxypentyle, 6-hydroxyhexyle ou 1-hydroxyhexyle ;
- le terme radical alcoxy renfermant de 1 à 6 atomes de carbone désigne par exemple un radical méthoxy, éthoxy, propoxy, isopropoxy, butoxy linéaire ou ramifié, pentoxy linéaire ou ramifié, hexyloxy linéaire ou ramifié ;
- le terme radical alcanoyloxy renfermant de 1 à 6 atomes de carbone désigne par exemple un radical formyloxy, acétoxy, propionyloxy, butyryloxy linéaire ou ramifié, pentanoyloxy linéaire ou ramifié, hexanoyloxy linéaire ou ramifié ;
- le terme radical alcoxycarbonyl renfermant de 2 à 6 atomes de carbone désigne par exemple le radical méthoxycarbonyle, éthoxycarbonyle, n-propoxycarbonyle, isopropoxycarbonyle, butoxycarbonyle linéaire ou ramifié, pentoxycarbonyle linéaire ou ramifié.

Les sels d'addition avec les acides minéraux ou organiques peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, alcanesulfoniques, tels que l'acide méthanesulfonique et arylsulfoniques, tels que l'acide benzène sulfonique.

Parmi les produits, objet de l'invention, on peut citer notamment les dérivés répondant à la formule (I) ci-dessus, ainsi que leurs sels d'addition avec les acides, caractérisés en ce que ladite formule (I) $R_1$ et $R_2$, identiques ou différents, représentent soit un atome d'hydrogène, soit un atome de chlore, soit un radical alcoyle renfermant de 1 à 6 atomes de carbone éventuellement substitué par un radical hydroxy, soit un radical alcoxy renfermant de 1 à 6 atomes de carbone, soit un radical alcanoyloxy renfermant de 1 à 6 atomes de carbone, soit un radical alcoxycarbonyle renfermant de 2 à 6 atomes de carbone, soit un radical hydroxy, soit un radical nitro, soit lorsque $R_1$ et $R_2$ sont portés par des atomes de carbone adjacents forment avec ceux-ci un radical phényle.

Parmi les produits, objet de l'invention, on peut citer particulièrement les dérivés répondant à la formule (I) ci-dessus ainsi que leurs sels d'addition avec les acides caractérisés en ce que dans ladite formule (I), $R_1$ représente un atome d'hydrogène et $R_2$ représente un atome d'hydrogène ou de chlore ou un radical méthyle, n-butyle, méthoxy, n-butoxy, 1-hydroxyhexyle, hydroxy, acétoxy ou nitro ou lorsque $R_1$ et $R_2$ sont

portés par 2 atomes de carbone adjacents, forment avec ceux-ci un radical phényle.

Parmi les produits, objet de l'invention, on peut citer tout particulièrement les dérivés de formule (I), dont les noms suivent :

- la 8-(1-naphthylthio) 5,6,7,8-tétrahydroquinoléine,
- la 8-(4-butylphénylthio) 5,6,7,8-tétrahydroquinoléine et son chlorhydrate,
- la 8-(2-naphthylthio) 5,6,7,8-tétrahydroquinoléine et son chlorhydrate,
- la 8-(4-méthylphénylthio) 5,6,7,8-tétrahydroquinoléine et son chlorhydrate.

L'invention a également pour objet un procédé de préparation des nouvelles 8-phénylthiotétrahydroquinoléines substituées telles que définies par la formule (I) ci-dessus, ainsi que de leurs sels, caractérisé en ce que l'on fait réagir un composé de formule (II) :

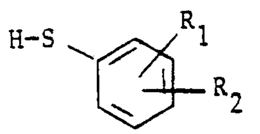

(II)

sous forme de sel dans laquelle $R_1$ et $R_2$ ont les significations déjà indiquées, avec un composé de formule (III) :

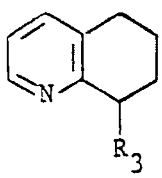

(III)

dans laquelle $R_3$ représente un atome d'halogène tel que le chlore, le brome ou l'iode, pour obtenir le composé de formule (I) que si désiré l'on salifie.

L'invention a aussi pour objet les variantes du procédé décrit ci-dessus, caractérisées en ce que : soit l'on fait réagir le produit de formule ($I_B$) :

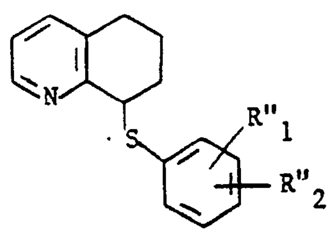

($I_B$)

sous forme de sel, formule dans laquelle $R''_1$ et $R''_2$ ont la signification indiquée pour $R_1$ et $R_2$ étant entendu que l'un au moins de $R''_1$ et $R''_2$ représente un radical hydroxy, avec un produit de formule (IV) :

R-X (IV)

dans laquelle R représente un radical alcoyle ou alcanoyle renfermant de 1 à 6 atomes de carbone et X représente un groupement facilement éliminable tel qu'un atome d'halogène ou un radical alcanoyloxy pour obtenir un produit de formule ($I_A$) :

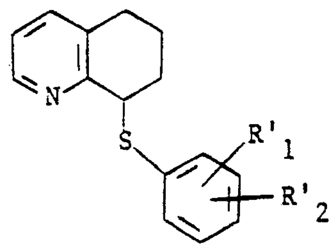

$(I_A)$

dans laquelle $R'_1$ et $R'_2$ ont la signification indiquée pour $R_1$ et $R_2$, étant entendu que l'un au moins de $R'_1$ et $R'_2$ représente un radical alcoxy ou alcanoyloxy renfermant de 1 à 6 atomes de carbone, que si désiré l'on salifie,

soit l'on estérifie le produit de formule $(I_D)$ :

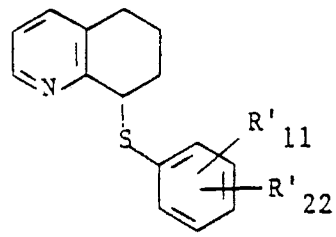

$(I_D)$

dans laquelle $R'_{11}$ et $R'_{22}$ ont la signification déjà indiquée pour $R_1$ et $R_2$ étant entendu que l'un au moins de $R'_{11}$ et $R'_{22}$ représente un radical carboxy pour obtenir un produit de formule $(I_C)$ :

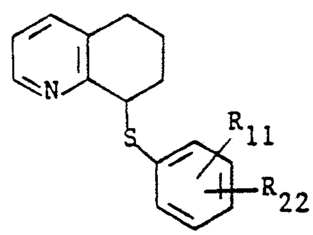

$(I_C)$

dans laquelle $R_{11}$ et $R_{22}$ ont la signification déjà indiquée pour $R_1$ et $R_2$ étant entendu que l'un au moins de $R_1$ et $R_2$ représente un radical alcoxycarbonyle renfermant de 2 à 6 atomes de carbone, que si désiré l'on salifie,

soit l'on effectue la nitration du produit de formule (I) correspondant répondant à la formule $(I_F)$ :

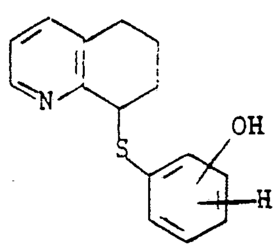

$(I_F)$

pour obtenir un produit de formule $(I_E)$ :

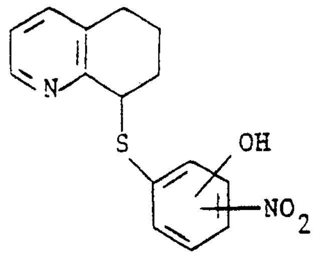

(I_E)

que si désiré, l'on salifie.

Dans des conditions préférentielles de mise en oeuvre de l'invention, le procédé de préparation et ses variantes ci-dessus décrit sont caractérisés en ce que :

- la réaction du produit de formule (II) avec le produit de formule (III) est effectuée au sein d'un solvant organique tel l'éther éthylique, le tétrahydrofuranne ou le diméthoxyméthane ;
- les sels des produits de formule (II) et ($I_B$) peuvent être préparés par traitement du produit de formule (II) ou ($I_B$) correspondant par un réactif capable de former un anion, par exemple un hydrure ou un carbonate de métal alcalin tel que l'hydrure de sodium ou le carbonate de potassium ;
- la réaction du produit de formule ($I_B$) avec le produit de formule (IV) est effectuée au sein d'un solvant organique tel que le tétrahydrofuranne ou le diméthylformamide ;
- lorsque le groupe éliminable X du produit de formule (IV) représente un radical alcanoyloxy renfermant de 1 à 6 atomes de carbone, l'anhydride résultant peut servir de réactif et de solvant ;
- l'estérification du produit de formule ($I_D$) est effectuée selon les méthodes usuelles, en traitant par exemple l'acide carboxylique de formule ($I_D$), ou un dérivé réactif de cet acide par un alcool de formule (V) :

  R'-OH (V)

  dans laquelle R' représente un radical alcoyle renfermant de 1 à 5 atomes de carbone, de préférence au sein d'un acide minéral tel que l'acide chlorhydrique et au reflux du milieu réactionnel ;
- l'alcool de formule (V) peut servir avantageusement de solvant ;
- la réaction de nitration est effectuée selon les méthodes usuelles, en traitant par exemple le produit de formule ($I_F$) par un système capable de produire "in situ" des ions $NO_2^+$, tel que le mélange nitrate de sodium, nitrate de lanthane en présence d'un acide minéral tel que l'acide chlorhydrique ;
- cette réaction est effectuée avantageusement au sein d'un solvant organique tel que l'éther éthylique, à basse température entre 0¤ et 5¤C par exemple.

Les produits de formule (I) présentent un caractère basique.

On peut avantageusement préparer les sels d'addition des produits de formule (I) en faisant réagir, en proportions sensiblement stoéchiométriques, un acide minéral ou organique avec lesdits produits de formuel (I). Les sels peuvent être préparés sans isoler les bases correspondantes.

Les produits, objet de la présente invention, possèdent de très intéressantes propriétés pharmacologiques ; ils sont doués notamment de remarquables propriétés inhibitrices de la 5-lipoxygénase et de la liaison de la leukotriène $D_4$ à ses récepteurs. En tant que tels, ils peuvent être utilisés pour leur propriétés anti-allergiques.

Ces propriétés justifient l'utilisation des nouvelles 8-phénylthiotétrahyquinoléines substituées de formule (I), ainsi que de leurs sels pharmaceutiquement acceptables, à titre de médicaments.

La présente invention a ainsi également pour objet l'application à titre de médicaments de nouvelles 8-phénylthiotétrahydroquinoléines substituées telles que définies par la formule générale (I) ainsi que de leurs sels d'addition avec les acides pharmaceutiquement acceptables.

Parmi les médicaments, objet de l'invention, on retient notamment les médicaments caractérisés en ce qu'ils sont constitués par les nouvelles 8-phénylthiotétrahydroquinoléines substituées répondant à la formule générale (I) dans laquelle $R_1$ et $R_2$, identiques ou différents, représentent soit un atome d'hydrogène, soit un atome de chlore, soit un radical alcoyle renfermant de 1 à 6 atomes de carbone éventuellement substitué par un radical hydroxy, soit un radical alcoxy renfermant de 1 à 6 atomes de carbone, soit un radical alcanoyloxy renfermant de 1 à 6 atomes de carbone, soit un radical alcoxycarbonyle renfermant de 2 à 6 atomes de carbone, soit un radical hydroxy, soit un radical nitro, soit lorsque $R_1$ et $R_2$ sont portés par des atomes de carbone adjacents forment avec ceux-ci un radical phényle, ainsi que par leurs sels d'addition avec les acides pharmaceutiquement acceptables.

Parmi les médicaments préférés de l'invention, on retient tout particulièrement ceux répondant à la formule (I) dans laquelle $R_1$ représente un atome d'hydrogène et $R_2$ représente un atome d'hydrogène ou de chlore ou un radical méthyle, n-butyle, méthoxy, n-butoxy, 1-hydroxyhexyle, hydroxy, acétoxy ou nitro ou lorsque $R_1$ et $R_2$ sont portés par 2 atomes de carbone adjacents, forment avec ceux-ci un radical phényle, ainsi que leurs sels d'addition avec les acides pharmaceutiquement acceptables.

Parmi les médicaments préférés de l'invention, on retient tout particulièrement :

- la 8-(1-naphthylthio) 5,6,7,8-tétrahydroquinoléine,
- la 8-(4-butylphénylthio) 5,6,7,8-tétrahydroquinoléine et son chlorhydrate,
- la 8-(2-naphthylthio) 5,6,7,8-tétrahydroquinoléine et son chlorhydrate,
- la 8-(4-méthylphénylthio) 5,6,7,8-tétrahydroquinoléine et son chlorhydrate.

Ces médicaments trouvent leur emploi dans le traitement des états asthmatiques allergiques et des bronchites d'origine allergique.

La dose usuelle, variable selon le produit utilisé, le sujet traité, et l'affection en cause, peut être par exemple de 0,1 mg à 200 mg par jour par voie orale.

L'invention a également pour objet les compositions pharmaceutiques, caractérisées en ce qu'elles renferment à titre de principe actif, l'un au moins des médicaments tels que définis précédemment.

A titre de médicaments, les dérivés répondant à la formule (I) et leurs sels d'addiction avec les acides pharmaceutiquement acceptables peuvent être incorporés dans des compositions pharmaceutiques destinées à la voie digestive, parentérale ou locale.

Ces compositions pharmaceutiques peuvent être, par exemple, solides ou liquides et se présenter sous les formes pharmaceutiques couramment utilisées en médecine humaine, comme par exemple les comprimés, simples ou dragéifiés, les gélules, les capsules, les granulés, les suppositoires, les aérosols, les crèmes, les pommades, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule (II) et de formule (III) sont décrits dans le littérature.

Les exemples suivants illustrent l'invention sans toutefois la limiter.

**Exemple 1 : Chlorhydrate de 8-(4-chlorophénylthio) 5,6,7,8-tétrahydroquinoléine.**

On ajoute sous agitation et sous atmosphère inerte 1,65 g d'hydrure de sodium dans une solution de 3,62 g de 4-chloro benzène thiol dans 36 cm3 de diméthylformamide. Après cessation du dégagement gazeux, on ajoute 4,8 g de chlorhydrate de 8-chloro 5,6,7,8-tétrahydroquinoléine et maintient sous agitation pendant 16 heures à température ambiante. On élimine le solvant sous pression réduite à température inférieure à 50¤C et sous atmosphère inerte, reprend le résidu dans une solution aqueuse d'hydroxyde de sodium 4N puis extrait à l'acétate d'éthyle. On lave la phase organique avec une solution aqueuse glacée d'hydroxyde de sodium puis de chlorure de sodium, sèche et évapore le solvant sous pression réduite, reprend le résidu dans l'éther et ajoute une solution de chlorure d'hydrogène dans l'éther. On obtient après filtration et cristallisation dans le mélange éther éthylique-éthanol, 5,13 g du chlorhydrate attendu.

**Exemple 2 : 4-(5,6,7,8-tétrahydroquinoléin-8-ylthio) benzonitrile.**

On agite pendant 16 heures sous atmosphère inerte à température ambiante 4,08 g de chlorhydrate 8-chloro 5,6,7,8-tétrahydroquinoléine, 3,38 g de 4-mercaptobenzonitrile et 6,91 g de carbonate de potassium dans 34 cm3 de diméthylformamide. On verse le mélange dans une solution aqueuse saturée en chlorure de sodium, extrait à l'acétate d'éthyle, lave avec une solution aqueuse glacée saturée en hydroxyde de sodium, puis à l'eau, sèche et élimine le solvant sous pression réduite. On obtient 4,72 g de produit attendu après recristallisation dans le mélange méthanol-dichlorométhane.

**Exemples 3 à 16:**

En utilisant une méthode analogue à celles décrites à l'exemple 1 ou 2 mais au départ des produits de formule (II) et (III) correspondants, on a préparé les produits des exemples 3 à 16. Le rendement, le point de fusion et les résultats de la microanalyse figurent dans le tableau I ci-après.

**Exemple 3 : Chlorhydrate de 8-(3-chlorophénylthio) 5,6,7,8-tétrahydroquinoléine.**

**Exemple 4 : Chlorhydrate de 8-(4-nitrophénylthio) 5,6,7,8-tétrahydroquinoléine.**
**Exemple 5 : Chlorhydrate de 8-(4-méthylphénylthio) 5,6,7,8-tétrahydroquinoléine.**
**Exemple 6 : 8-(1-naphthylthio) 5,6,7,8-tétrahydroquinoléine.**
**Exemple 7 : 8-(4-aminophénylthio) 5,6,7,8-tétrahydroquinoléine.**
**Exemple 8 : 8-(4-hydroxyphénylthio) 5,6,7,8-tétrahydroquinoléine.**
**Exemple 9 : Chlorhydrate de 8-(2-naphthylthio) 5,6,7,8-tétrahydroquinoléine.**
**Exemple 10 : Chlorhydrate de 8-(4-n-butylphénylthio) 5,6,7,8-tétrahydroquinoléine.**
**Exemple 11 : 8-[3-(1-hydroxyhexylphénylthio) 5,6,7,8-tétrahydroquinoléine.**
**Exemple 12 : 4-(5,6,7,8-tétrahydroquinoléin-8-ylthio) benzènesulfonamide.**
**Exemple 13 : 8-(phénylthio) 5,6,7,8-tétrahydroquinoléine.**
**Exemple 14 : Chlorhydrate de 8-(4-méthoxyphénylthio) 5,6,7,8-tétrahydroquinoléine.**
**Exemple 15 : Chlorhydrate de 8-(4-éthylphénylthio) 5,6,7,8-tétrahydroquinoléine.**
**Exemple 16 : Acide 4-(5,6,7,8-tétrahydroquinoléin-8-ylthio) benzoïque.**

**Exemple 17 : Chlorhydrate de 8-(4-n-butoxyphénylthio) 5,6,7,8-tétrahydroquinoléine.**

On ajoute sous atmosphère inerte, 0,72 g d'hydrure de sodium dans une solution de 5,15 g de 8-(4-hydroxyphénylthio) 5,6,7,8,-tétrahydroquinoléine dans 26 cm3 de diméthylformamide. Après cessation du dégagement gazeux, on ajoute à 0¤C, 3,29 g de n-bromobutane puis maintient 16 heures sous agitation à température ambiante. On verse le milieu réactionnel dans l'eau glacée, extrait à l'éther, traite le résidu par une solution de chlorure d'hydrogène dans l'éther et obtient après recristallisation dans le mélange éther éthylique-éthanol, 4,89 g du chlorhydrate attendu.

**Exemple 18 : Chlorhydrate de 8-(4-éthoxyphénylthio) 5,6,7,8,-tétrahydroquinoléine.**

On opère comme à l'exemple 17 en remplaçant le n-bromobutane par du bromoéthane.

**Exemple 19 : Chlorhydrate de 8-(4-acétoxyphénylthio) 5,6,7,8-tétrahydroquinoléine.**

On chauffe au reflux pendant 2 heures, 4 g de 8-(4-hydroxyphénylthio) 5,6,7 8-tétrahydroquinoléine dans 40 cm3 d'anhydride acétique. On élimine le réactif sous pression réduite, chromatographie le résidu sur silice (éluant : dichlorométhane-acétate d'éthyle 95-5) puis traite par une solution de chlorure d'hydrogène dans l'éther et obtient 3,4 g de produit attendu.

**Exemple 20 : Chlorhydrate de méthyl 4-(5,6,7,8,-tétrahydroquinoléin-8-ylthio) benzoate.**

On chauffe au reflux pendant 2 heures, 4,24 g d'acide 4-(5,6,7,8-tétrahydroquinoléin-8-ylthio) benzoïque préparé à l'exemple 16 dans 30 cm3 de méthanol saturé en chlorure d'hydrogène, élimine le solvant sous pression réduite, reprend le résidu dans l'eau glacée, alcalinise à l'aide de carbonate de sodium puis extrait à l'éther. Après évaporation de l'éther, on traite le résidu avec une solution de chlorure d'hydrogène dans l'éther et obtient 2,9 g de produit attendu.

**Exemple 21 : 8-(4-hydroxy-3-nitrophénylthio) 5,6,7,8,-tétrahydroquinoléine.**

On ajoute à 0¤/5¤C une solution de 15 g de 8-(4-hydroxyphénylthio) 5,6,7,8-tétrahydroquinoléine dans 90 cm3 d'éther dans une solution comprenant 4,96 g de nitrate de sodium et 0,25 g de nitrate de lanthane dans 60 cm3 d'acide chlorhydrique et maintient sous agitation et atmosphère inerte pendant 24 heures. On ajoute de l'eau, extrait au chloroforme, élimine le solvant, chromatographie le résidu sur silice (éluant : dichlorométhane-acétate d'éthyle 9-1) et obtient 5,6 g de produit attendu.

Les rendements, points de fusion et la microanalyse des produits des exemples 1 à 21 sont indiqués dans le tableau I ci-après.

TABLEAU I

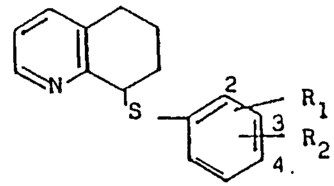

| Exemple | $R_1$ | $R_2$ | Rendt % | F (°C) | Formule | Calculé/Trouvé C% | H% | N% | S% |
|---|---|---|---|---|---|---|---|---|---|
| 1 | H | 4-Cl | 82 | 130-52 | $C_{15}H_{15}Cl_2NS$* | 57.70<br>57.57 | 4.84<br>4.90 | 4.49<br>4.49 | |
| 2 | H | 4-CN | 87 | 117-20 | $C_{16}H_{14}N_2S$ | 72.15<br>72.24 | 5.30<br>5.35 | 10.52<br>10.52 | |
| 3 | 3-Cl | H | 79 | 148-60 | $C_{15}H_{15}Cl_2NS$* | 57.70<br>57.91 | 4.84<br>4.87 | 4.49<br>4.57 | |
| 4 | H | $4-NO_2$ | 80 | 154-60 | $C_{15}H_{15}ClN_2O_2S$* | 55.81<br>55.59 | 4.68<br>4.71 | 8.68<br>8.68 | |
| 5 | H | $4-CH_3$ | 75 | 157-71 | $C_{16}H_{18}ClNS$* | 65.85<br>65.45 | 6.22<br>6.23 | 4.80<br>4.83 | |
| 6 | $2,3-C_4H_4$ | | 63 | 96-7 | $C_{19}H_{17}NS$ | 78.31<br>77.98 | 5.88<br>5.94 | 4.81<br>4.74 | |
| 7 | H | $4-NH_2$ | 41 | 129-31 | $C_{15}H_{16}N_2S$ | 70.28<br>70.13 | 6.29<br>6.30 | 10.93<br>10.87 | |
| 8 | H | 4-OH | 66 | 189-90 | $C_{15}H_{15}NOS$ | 70.01<br>70.05 | 5.88<br>5.90 | 5.44<br>5.45 | |
| 9 | $3,4-C_4H_4$ | | 84 | 151-160 | $C_{19}H_{18}ClNS$* | 69.60<br>70.41 | 5.53<br>5.61 | 4.27<br>4.32 | |
| 10 | H | 4-n-butyl | 87 | 127-46 | $C_{19}H_{24}ClNS$* | 68.34<br>68.31 | 7.24<br>7.29 | 4.20<br>4.19 | |

* Chlorhydrate

TABLEAU I

| Exemple | $R_1$ | $R_2$ | Rendt % | F (°C) | Formule | Calculé/Trouvé C% | H% | N% | S% |
|---|---|---|---|---|---|---|---|---|---|
| 11 | 3-(1-hydroxy hexyl) | H | 82 | 54-63 | $C_{21}H_{27}NOS$ | 73.86<br>.73.53 | 7.97<br>8.03 | 4.10<br>4.07 | 9.39<br>9.36 |
| 12 | H | 4-$SO_2NH_2$ | 29 | 184-7 | $C_{15}H_{16}N_2O_2S_2$ | 56.23<br>56.19 | 5.03<br>5.04 | 8.74<br>8.76 | |
| 13 | H | H | 15 | (oil) | $C_{15}H_{15}NS$ | 74.64<br>74.44 | 6.28<br>6.37 | 5.80<br>5.69 | 13.28<br>13.10 |
| 14 | H | 4-methoxy | 64 | 127-42 | $C_{16}H_{18}ClNOS$* 1/4$H_2O$ | 61.53<br>61.57 | 5.97<br>5.84 | 4.49<br>4.49 | |
| 15 | H | 4-ethyl | 73 | 163-7 | $C_{17}H_{20}ClNS$* | 66.75<br>66.54 | 6.59<br>6.70 | 4.58<br>4.40 | |
| 16 | H | 4-COOH | 45 | 199-204 | $C_{16}H_{15}NO_2S$ | 67.34<br>67.14 | 5.30<br>5.32 | 4.91<br>4.82 | |
| 17 | H | 4-n-butoxy | 70 | 121-8 | $C_{19}H_{24}ClNOS$* | 65.22<br>64.83 | 6.91<br>6.85 | 4.00<br>3.96 | |
| 18 | H | 4-OEt | 71 | 150-4 | $C_{17}H_{20}ClNOS$* | 63.44<br>63.10 | 6.26<br>6.25 | 4.35<br>4.29 | |
| 19 | H | 4-$OCOCH_3$ | 49 | 98-102 | $C_{17}H_{18}ClNO_2S.H_2O$* | 57.90<br>57.82 | 5.70<br>5.74 | 3.96<br>3.82 | |
| 20 | H | 4-$COOCH_3$ | 62 | 150-5 | $C_{17}H_{18}ClNO_2S$* | 60.79<br>60.49 | 5.40<br>5.47 | 4.17<br>4.09 | |
| 21 | 3-$NO_2$ | 4-OH | 20 | 122-4 | $C_{15}H_{14}N_2O_3S$ | 59.58<br>59.48 | 4.72<br>4.67 | 9.27<br>9.21 | |

* Chlorhydrate

**Exemple 22:**

On a préparé des comprimés répondant à la formulation suivante :

- Produit de l'exemple 6 ........................................ 20 mg
- Excipient q.s. pour un comprimé terminé à ................. 100 mg.

(Détail de l'excipient : lactose, amidon, talc, stéarate de magnésium).

**Exemple 23:**

On a préparé des comprimés répondant à la formulation suivante :

- Produit de l'exemple 10 ...................................... 20 mg
- Excipient q.s. pour un comprimé terminé à ................. 100 mg.

(Détail de l'excipient : lactose, amidon, talc, stéarate de magnésium).

**Exemple 24:**

On a préparé un aérosol délivrant par dose :

- Produit de l'exemple 9 ........................................ 2 mg
- Emulsifiant ................................................... 0,15 mg
- Propulseur .................................................... 50 mg.

**ETUDE BIOCHIMIQUE**

**5-Lipox**

L'inhibition de l'ionophore $Ca^{++}$ (A 23187) provoque la libération de produits 5-lipoxygénase (Leukotriene $B_4$ et 5-HETE) de l'acide /14c/arachidonique des neutrophiles péritonéales pré-marqués du rat.

On a utilisé la méthode de Ahnfelt-Ronne, I. et Arrigoni-Martelli, E. Biochemical Pharmacology, Vol. 31, N¤ 16, p. 2619-2624 (1982) modifiée. Les valeurs indiquées sont des concentrations micromolaires du composé testé provoquant 50% d'inhibition par rapport à la réponse du témoin déterminées graphiquement d'après les courbes de réponse par dose.

**Récepteur $LTD_4$**

Etude de l'inhibition de la liaison spécifique du /$^3$H/ $LTD_4$ sur une préparation de membrane de tissu de poumon de cobaye. L'évaluation a été effectuée par une méthode modifiée de Bruns, R.F. Thomsen, W.J. et Pugsley, T.A. Life Sciences, Vol. 33, p. 645-653, (1983). Les valeurs indiquées sont des concentrations micromolaires du composé testé provoquant 50% d'inhibition de la liaison spécifique déterminée graphiquement d'après les courbes de réponse par dose.

Les résultats de ces tests sont donnés dans le tableau II.

TABLEAU II

| Exemple | 5-LIPOX | récepteur $LTD_4$ |
|---|---|---|
| 1 | 4,2 | 22,4 |
| 2 | 31 | |
| 4 | 12,6 | 26,3 |
| 5 | 3,0 | 23,4 |
| 6 | 1,7 | 52,5 |
| 7 | 25 | >100 |
| 8 | 14,1 | 6,3 |
| 9 | 2,3 | |
| 10 | 1,9 | |
| 11 | 5,6 | |
| 12 | 26 | |
| 14 | 13,2 | |
| 15 | 4,0 | |
| 16 | >100 | |
| 17 | 3,7 | |
| 18 | 11,2 | |
| 19 | 45 | |
| 20 | 18,6 | |
| 21 | 33 | |

## Revendications

**1.** 8-phénylthiotétrahydroquinoléines substituées répondant à la formule (I) :

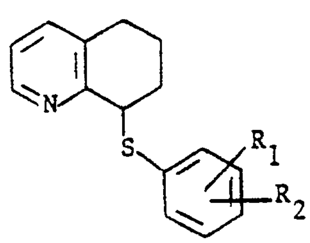

(I)

dans laquelle $R_1$ et $R_2$, identiques ou différents, représentent soit un atome d'hydrogène, soit un atome d'halogène, soit un radical alcoyle renfermant de 1 à 6 atomes de carbone éventuellement substitué par un radical hydroxyle, soit un radical alcoxyle renfermant de 1 à 6 atomes de carbone, soit un radical alcanoyloxy renfermant de 1 à 6 atomes de carbone, soit un radical alcoxycarbonyl renfermant de 2 à 6 atomes de carbone, soit un radical hydroxy, nitro, amino, carboxy, cyano ou aminosulfonyl, soit lorsque $R_1$ et $R_2$ sont portés par des atomes de carbone adjacents, forment avec ceux-ci un radical phényle, ainsi que leurs sels d'addition avec les acides.

2. 8-phénylthiotétrahydroquinoléines substituées telles que définies à la revendication 1, et leurs sels d'addition avec les acides, caractérisées en ce que dans ladite formule (I), $R_1$ et $R_2$, identiques ou différents, représentent soit un atome d'hydrogène, soit un atome de chlore, soit un radical alcoyle renfermant de 1 à 6 atomes de carbone éventuellement substitué par un radical hydroxy, soit un radical alcoxy renfermant de 1 à 6 atomes de carbone, soit un radical alcanoyloxy renfermant de 1 à 6 atomes de carbone, soit un radical alcoxy-carbonyle renfermant de 2 à 6 atomes de carbone, soit un radical hydroxy, soit un radical nitro, soit lorsque $R_1$ et $R_2$ sont portés par des atomes de carbone adjacents forment avec ceux-ci un radical phényle.

3. 8-phénylthiotétrahydroquinoléines substituées telles que définies à la revendication 1 ou 2, et leurs sels d'addition avec les acides, caractérisés en ce que dans ladite formule (I), $R_1$ représente un atome d'hydrogène et $R_2$ représente un atome d'hydrogène ou de chlore ou un radical méthyle, n-butyle, méthoxy, n-butoxy, 1-hydroxyhexyle, hydroxy, acétoxy ou nitro ou lorsque $R_1$ et $R_2$ sont portés par 2 atomes de carbone adjacents, forment avec ceux-ci un radical phényle.

4. L'un quelconque des dérivés répondant à la formule (I) de la revendication 1, dont les noms suivent :
- la 8-(1-naphthylthio) 5,6,7,8-tétrahydroquinoléine,
- la 8-(4-butylphénylthio) 5,6,7,8-tétrahydroquinoléine et son chlorhydrate,
- la 8-(2-naphthylthio) 5,6,7,8-tétrahydroquinoléine et son chlorhydrate,
- la 8-(4-méthylphénylthio) 5,6,7,8-tétrahydroquinoléine et son chlorhydrate.

5. Procédé de préparation des nouvelles 8-phénylthiotétrahydroquinoléines substituées telles que définies par la formule (I) de la revendication 1, ainsi que de leurs sels, caractérisé en ce que l'on fait réagir un composé de formule (II) :

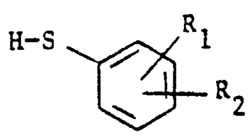

(II)

sous forme de sel, formule dans laquelle $R_1$ et $R_2$ ont les significations déjà indiquées, avec un composé de formule (III) :

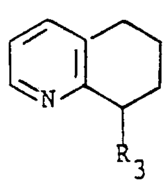

(III)

dans laquelle $R_3$ représente un atome d'halogène tel que le chlore, le brome ou l'iode, pour obtenir un composé de formule (I) que si désiré l'on salifie.

6. Variante du procédé de préparation selon la revendication 5, caractérisée en ce que l'on fait réagir le produit de formule ($I_B$)

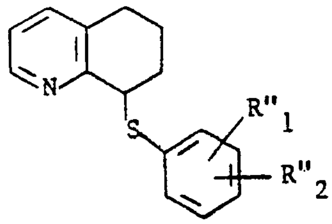

$(I_B)$

sous forme de sel, formule dans laquelle $R''_1$ et $R''_2$ ont la signification indiquée pour $R_1$ et $R_2$ étant entendu que l'un au moins de $R''_1$ et $R''_2$ représente un radical hydroxy, avec un produit de formule (IV) :

R-X (IV)

dans laquelle R représente un radical alcoyle ou alcanoyle renfermant de 1 à 6 atomes de carbone et X représente un groupement facilement éliminable tel qu'un atome d'halogène ou un radical alcanoyloxy pour obtenir un produit de formule $(I_A)$ :

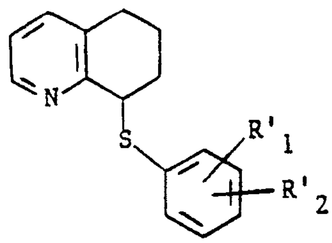

$(I_A)$

dans laquelle $R'_1$ et $R'_2$ ont la signification indiquée pour $R_1$ et $R_2$, étant entendu que l'un au moins de $R'_1$ et $R'_2$ représente un radical alcoxy ou alcanoyloxy renfermant de 1 à 6 atomes de carbone, que si désiré l'on salifie.

7. Variante du procédé de préparation selon la revendication 5, caractérisée en ce que l'on estérifie le produit de formule $(I_D)$ :

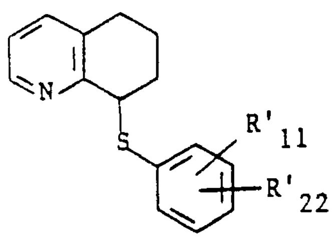

$(I_D)$

dans laquelle $R'_{11}$ et $R'_{22}$ ont la signification déjà indiquée pour $R_1$ et $R_2$ étant entendu que l'un au moins de $R'_{11}$ et $R'_{22}$ représente un radical carboxy pour obtenir un produit de formule $(I_C)$ :

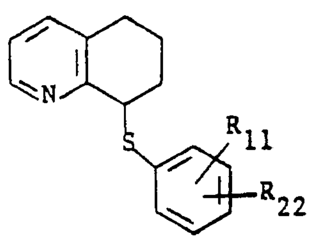

$(I_C)$

dans laquelle $R_{11}$ et $R_{22}$ ont la signification déjà indiquée pour $R_1$ et $R_2$ étant entendu que l'un au moins de $R_1$ et $R_2$ représente un radical alcoxycarbonyle renfermant de 2 à 6 atomes de carbone, que

si désiré l'on salifie.

8. Variante du procédé de préparation selon la revendioation 5, caractérisée en ce que l'on effectue la nitration du produit de formule ($I_F$ ) :

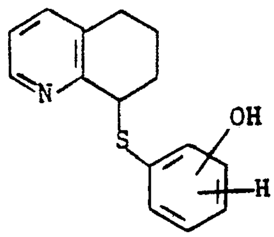

($I_F$)

pour obtenir un produit de formule ($I_E$) :

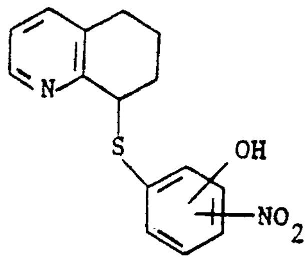

($I_E$)

que si désiré, l'on salifie.

9. Procédé de préparation ou ses variantes selon l'une des revendications 5 à 8, caractérisés en ce que :
   - la réaction du produit de formule (II) avec le produit de formule (III) est effectuée au sein d'un solvant organique tel l'éther éthylique, le tétrahydrofuranne ou le diméthoxyméthane ;
   - les sels des produits de formule (II) et ($I_B$) peuvent être préparés par traitement du produit de formule (II) ou ($I_B$) correspondant par un réactif capable de former un anion, par exemple un hydrure ou un carbonate de métal alcalin tel que l'hydrure de sodium ou le carbonate de potassium ;
   - la réaction du produit de formule ($I_B$) avec le produit de formule (IV) est effectuée au sein d'un solvant organique tel que le tétrahydrofuranne ou le diméthylformamide ;
   - lorsque le groupe éliminable X du produit de formule (IV) représente un radical alcanoyloxy renfermant de 1 à 6 atomes de carbone, l'anhydride résultant peut servir de réactif et de solvant ;
   - l'estérification du produit de formule ($I_D$) est effectuée selon les méthodes usuelles, en traitant par exemple l'acide carboxylique de formule ($I_D$), ou un dérivé réactif de cet acide par un alcool de formule (V) :

     R'-OH (V)

     dans laquelle R' représente un radical alcoyle renfermant de 1 à 5 atomes de carbone, de préférence au sein d'un acide minéral tel que l'acide chlorhydrique et au reflux du milieu réactionnel ;
   - l'alcool de formule (V) peut servir avantageusement de solvant ;
   - la réaction de nitration est effectuée selon les méthodes usuelles, en traitant par exemple le produit de formule ($I_F$) par un système capable de produire "in situ" des ions $NO_2^+$, tel que le mélange nitrate de sodium, nitrate de lauthane en présence d'un acide minéral tel que l'acide chlorhydrique ;
   - cette réaction est effectuée avantageusement au sein d'un solvant organique tel que l'éther éthylique, à basse température entre 0¤ et 5¤C par exemple.

10. Médicaments, caractérisés en ce qu'ils sont constitués par les nouvelles 8-phénylthiotétrahydroquinoléines substituées telles que définies par la formule (I) de la revendication 1, ainsi que leurs sels

d'addition avec les acides pharmaceutiquement acceptables.

**11.** Médicaments, caractérisés en ce qu'ils sont constitués par les nouvelles 8-phénylthiotétrahydroquinoléines substituées telles que définies à l'une quelconque des revendications 2 ou 3, ainsi que leurs sels d'addition avec les acides pharmaceutiquement acceptables.

**12.** Médicaments, caractérisés en ce qu'ils sont constitués par les nouvelles 8-phénylthiotétrahydroquinoléines substituées telles que définies à la revendication 4.

**13.** Compositions pharmaceutiques, caractérisées en ce qu'elles renferment à titre de principe actif, l'un au moins des médicaments tels que définis à l'une quelconque des revendications 10 à 12.

Revendications pour les Etats contractants suivants : AT, ES, GR

**1.** Procédé de préparation des 8-phénylthiotétrahydroquinoléines substituées répondant à la formule (I) :

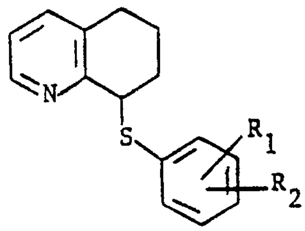

(I)

dans laquelle $R_1$ et $R_2$, identiques ou différents, représentent soit un atome d'hydrogène, soit un atome d'halogène, soit un radical alcoyle renfermant de 1 à 6 atomes de carbone éventuellement substitué par un radical hydroxyle, soit un radical alcoxyle renfermant de 1 à 6 atomes de carbone, soit un radical alcanoyloxy renfermant de 1 à 6 atomes de carbone, soit un radical alcoxycarbonyl renfermant de 2 à 6 atomes de carbone, soit un radical hydroxy, nitro, amino, carboxy, cyano ou aminosulfonyl, soit lorsque $R_1$ et $R_2$ sont portés par des atomes de carbone adjacents, forment avec ceux-ci un radical phényle, ainsi que leurs sels d'addition avec les acides, caractérisé en ce que l'on fait réagir un composé de formule (II) :

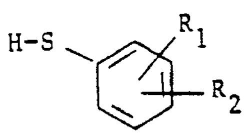

(II)

sous forme de sel, formule dans laquelle $R_1$ et $R_2$ ont les significations déjà indiquées, avec un composé de formule (III) :

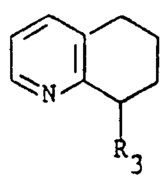

(III)

dans laquelle $R_3$ représente un atome d'halogène tel que le chlore, le brome ou l'iode, pour obtenir un composé de formule (I) que si désiré l'on salifie

**2.** Variante du procédé selon la revendication 1, caractérisée en ce que l'on fait réagir le produit de formule ($I_B$) :

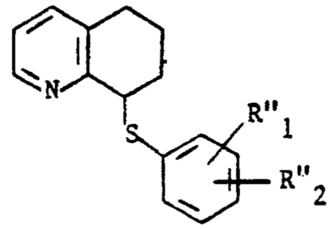

($I_B$)

sous forme de sel, formule dans laquelle $R''_1$ et $R''_2$ ont la signification indiquée pour $R_1$ et $R_2$ étant entendu que l'un au moins de $R''_1$ et $R''_2$ représente un radical hydroxy, avec un produit de formule (IV) :

R-X (IV)

dans laquelle R représente un radical alcoyle ou alcanoyle renfermant de 1 à 6 atomes de carbone et X représente un groupement facilement éliminable tel qu'un atome d'halogène ou un radical alcanoyloxy pour obtenir un produit de formule ($I_A$) :

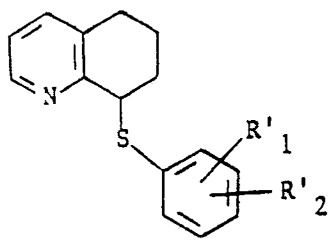

($I_A$)

dans laquelle $R'_1$ et $R'_2$ ont la signification indiquée pour $R_1$ et $R_2$, étant entendu que l'un au moins de $R'_1$ et $R'_2$ représente un radical alcoxy ou alcanoyloxy renfermant de 1 à 6 atomes de carbone, que si désiré l'on salifie.

3. Variante du procédé selon la revendication 1, caractérisée en ce que l'on estérifie le produit de formule ($I_D$) :

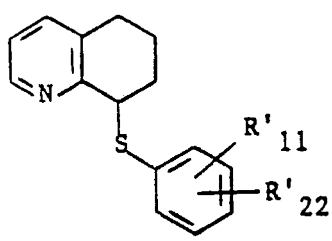

($I_D$)

dans laquelle $R'_{11}$ et $R'_{22}$ ont la signification déjà indiquée pour $R_1$ et $R_2$ étant entendu que l'un au moins de $R'_{11}$ et $R'_{22}$ représente un radical carboxy pour obtenir un produit de formule ($I_C$) :

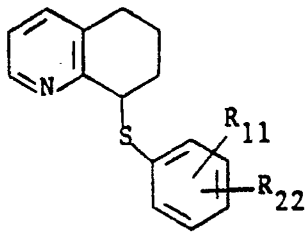

($I_C$)

dans laquelle $R_{11}$ et $R_{22}$ ont la signification déjà indiquée pour $R_1$ et $R_2$ étant entendu que l'un au moins de $R_1$ et $R_2$ représente un radical alcoxycarbonyle renfermant de 2 à 6 atomes de carbone, que

si désiré l'on salifie

4. Variante du procédé selon la revendication 1 caractérisée en que l'on effectue la nitration du produit de formule ($I_F$) :

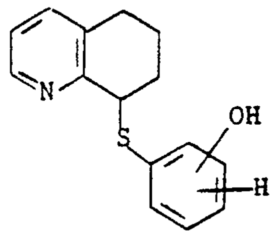

($I_F$)

pour obtenir un produit de formule ($I_E$) :

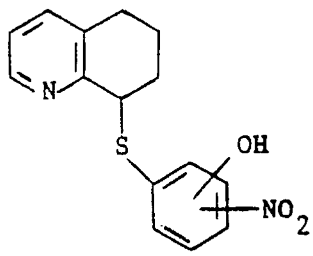

($I_E$)

que si désiré, l'on salifie.

5. Procédé de préparation ou ses variantes selon l'une des revendications 1 à 4, caractérisé en ce que :
- la réaction du produit de formule (II) avec le produit de formule (III) est effectuée au sein d'un solvant organique tel l'éther éthylique, le tétrahydrofuranne ou le diméthoxyméthane ;
- les sels des produits de formule (II) et ($I_B$) peuvent être préparés par traitement du produit de formule (II) ou ($I_B$) correspondant par un réactif capable de former un anion, par exemple un hydrure ou un carbonate de métal alcalin tel que l'hydrure de sodium ou le carbonate de potassium ;
- la réaction du produit de formule ($I_B$) avec le produit de formule (IV) est effectuée au sein d'un solvant organique tel que le tétrahydrofuranne ou le diméthylformamide ;
- lorsque le groupe éliminable X du produit de formule (IV) représente un radical alcanoyloxy renfermant de 1 à 6 atomes de carbone, l'anhydride résultant peut servir de réactif et de solvant ;
- l'estérification du produit de formule ($I_D$) est effectuée selon les méthodes usuelles, en traitant par exemple l'acide carboxylique de formule ($I_D$), ou un dérivé réactif de cet acide par un alcool de formule (V) :

  R'-OH (V)

  dans laquelle R' représente un radical alcoyle renfermant de 1 à 5 atomes de carbone, de préférence au sein d'un acide minéral tel que l'acide chlorhydrique et au reflux du milieu réactionnel ;
- l'alcool de formule (V) peut servir avantageusement de solvant ;
- la réaction de nitration est effectuée selon les méthodes usuelles, en traitant par exemple le produit de formule ($I_F$) par un système capable de produire "in situ" des ions $NO_2^+$, tel que le mélange nitrate de sodium, nitrate de lauthane en présence d'un acide minéral tel que l'acide chlorhydrique ;
- cette réaction est effectuée avantageusement au sein d'un solvant organique tel que l'éther éthylique, à basse température entre 0¤ et 5¤C par exemple.

6. Procédé de préparation selon la revendication 1 ou 5, caractérisé en ce que l'on utilise au départ un produit de formule (II) dans laquelle $R_1$ et $R_2$, identiques ou différents, représentent soit un atome

d'hydrogène, soit un atome de chlore, soit un radical alcoyle renfermant de 1 à 6 atomes de carbone éventuellement substitué par un radical hydroxy, soit un radical alcoxy renfermant de 1 à 6 atomes de carbone, soit un radical alcanoyloxy renfermant de 1 à 6 atomes de carbone, soit un radical alcoxy-carbonyle renfermant de 2 à 6 atomes de carbone, soit un radical hydroxy, soit un radical nitro, soit lorsque $R_1$ et $R_2$ sont portés par des atomes de carbone adjacents forment avec ceux-ci un radical phényle.

7. Procédé de préparation selon la revendication 1 ou 5, caractérisé en ce que l'on utilise au départ un produit de formule (II) dans laquelle $R_1$ représente un atome d'hydrogène et $R_2$ représente un atome d'hydrogène ou de chlore ou un radical méthyle, n-butyle, méthoxy, n-butoxy, 1-hydroxyhexyle, hydroxy, acétoxy ou nitro ou lorsque $R_1$ et $R_2$ sont portés par 2 atomes de carbone adjacents, forment avec ceux-ci un radical phényle.

8. Procédé de préparation selon l'une quelconque des revendications 1 à 7 4, caractérisé en ce que l'on prépare :
   - la 8-(1-naphthylthio) 5,6,7,8-tétrahydroquinoléine,
   - la 8-(4-butylphénylthio) 5,6,7,8-tétrahydroquinoléine et son chlorhydrate,
   - la 8-(2-naphthylthio) 5,6,7,8-tétrahydroquinoléine et son chlorhydrate,
   - la 8-(4-méthylphénylthio) 5,6,7,8-tétrahydroquinoléine et son chlorhydrate.

9. Procédé de préparation de compositions pharmaceutiques, caractérisé en ce que l'on met comme principe actif l'un au moins des produits de formule (I) tel qu'obtenu à la revendication 1 ou l'un au moins de ses sels pharmaceutiquement acceptables sous une forme adaptée à l'usage thérapeutique.

10. Procédé de préparation de compositions pharmaceutiques, caractérisé en ce que l'on met comme principe actif l'un au moins des produits de formule (I) tel qu'obtenu à l'une quelconque des revendications 2 à 7 ou l'un au moins de ses sels pharmaceutiquement acceptables sous une forme adaptée à l'usage thérapeutique.

11. Procédé de préparation de compositions pharmaceutiques, caractérisé en ce que l'on met comme principe actif l'un au moins des produits de formule (I) tel qu'obtenu à la revendication 8 ou l'un au moins de ses sels pharmaceutiquement acceptables sous une forme adaptée à l'usage thérapeutique.

**Claims**

1. Substituted 8-phenylthiotetrahydroquinolines corresponding to formula (I):

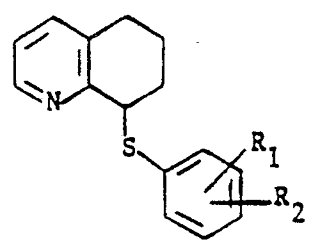

(I)

in which $R_1$ and $R_2$, identical or different, represent either a hydrogen atom, or a halogen atom, or an alkyl radical containing 1 to 6 carbon atoms optionally substituted by a hydroxyl radical, or an alkoxyl radical containing 1 to 6 carbon atoms, or an alkanoyloxy radical containing 1 to 6 carbon atoms, or an alkoxycarbonyl radical containing 2 to 6 carbon atoms, or a hydroxy, nitro, amino, carboxy, cyano or aminosulphonyl radical, or when $R_1$ and $R_2$ are carried by the adjacent carbon atoms, form with these a phenyl radical, as well as their addition salts with acids.

2. Substituted 8-phenylthiotetrahydroquinolines as defined in claim 1, as well as their addition salts with acids, characterized in that in said formula (I), $R_1$ and $R_2$, identical of different, represent either a hydrogen atom, or a chlorine atom, or an alkyl radical containing 1 to 6 carbon atoms optionally substituted by a hydroxy radical, or an alkoxy radical containing 1 to 6 carbon atoms, or an alkanoyloxy

radical containing 1 to 6 carbon atoms, or an alkoxy-carbonyl radical containing 2 to 6 carbon atoms, or a hydroxy radical, or a nitro radical, or when $R_1$ and $R_2$ are carried by the adjacent carbon atoms form with these a phenyl radical.

**3.** Substituted 8-phenylthiotetrahydroquinolines as defined in claim 1 or 2, and their addition salts with acids, characterized in that in said formula (I), $R_1$ represents a hydrogen atom and $R_2$ represents a hydrogen or a chlorine atom or a methyl, n-butyl, methoxy, n-butoxy, 1-hydroxyhexyl, hydroxy, acetoxy or nitro radical or when $R_1$ and $R_2$ are carried by the two adjacent carbon atoms form with these a phenyl radical.

**4.** Any one of the derivatives corresponding to formula (I) of claim 1, the names of which follow:
- 8-(1-naphthylthio)-5,6,7,8-tetrahydroquinoline,
- 8-(4-butylphenylthio)-5,6,7,8-tetrahydroquinoline and its hydrochloride,
- 8-(2-naphthylthio)-5,6,7,8-tetrahydroquinoline and its hydrochloride,
- 8-(4-methylphenylthio)-5,6,7,8-tetrahydroquinoline and its hydrochloride.

**5.** Preparation process for new substituted 8-phenylthiotetrahydroquinolines as defined by formula (1) of claim 1, as well as their salts, characterized in that a compound of formula (II):

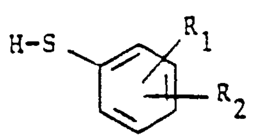

(II)

in the form of the salt, in which formula $R_1$ and $R_2$ have the meaning already indicated, is reacted with a compound of formula (III):

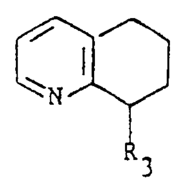

(III)

in which $R_3$ represents a halogen atom such as chlorine, bromine or iodine, in order to obtain a compound of formula (I) which, if desired, is salified.

**6.** Variant of the preparation process according to claim 5, characterized in that the product of formula ($I_B$):

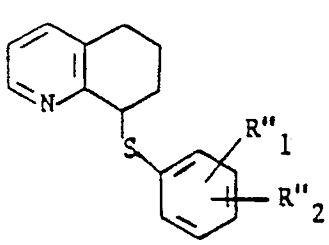

($I_B$)

in the form of the salt, in which formula $R"_1$ and $R"_2$ have the meaning indicated for $R_1$ and $R_2$ it being understood that at least one of $R"_1$ and $R"_2$ represents a hydroxy radical, is reacted with a product of formula (IV) :

R-X (IV)

in which R represents an alkyl or alkanoyl radical containing 1 to 6 carbon atoms and X represents an easily eliminable group such as a halogen atom or an alkanoyloxy radical in order to obtain a product of

formula ($I_A$) :

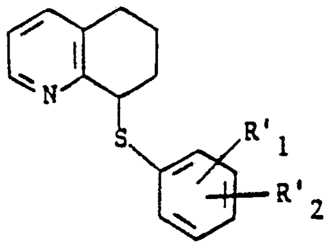

($I_A$)

in which $R'_1$ and $R'_2$ have the meaning indicated for $R_1$ and $R_2$, it being understood that at least one of $R'_1$ and $R'_2$ represents an alkoxy or alkanoyloxy radical containing 1 to 6 carbon atoms, which, if desired, is salified.

7. Variant of the preparation process according to claim 5, characterized in that the product of formula ($I_D$):

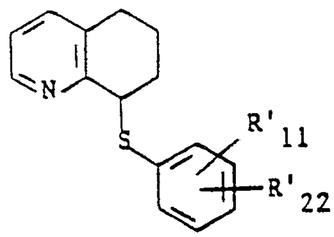

($I_D$)

in which $R'_{11}$ and $R'_{22}$ have the meaning already indicated for $R_1$ and $R_2$, it being understood that at least one of $R'_{11}$ and $R'_{22}$ represents a carboxy radical, is esterified in order to obtain a product of formula ($I_C$):

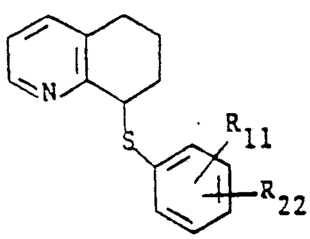

($I_C$)

in which $R_{11}$ and $R_{22}$ have the meaning already indicated for $R_1$ and $R_2$ it being understood that at least one of $R_1$ and $R_2$ represents an alkoxycarbonyl radical containing 2 to 6 carbon atoms, which, if desired, is salified.

8. Variant of the preparation process according to claim 5, characterized in that the nitration of the product of formula ($I_F$):

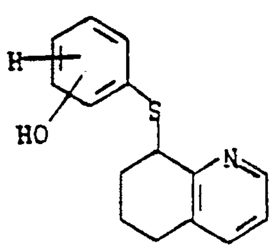

($I_F$)

is carried out in order to obtain a product of formula ($I_E$):

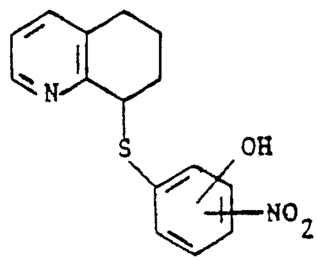

$(I_E)$

which, if desired, is salified.

**9.** Preparation process or its variants according to one of claims 5 to 8, characterized in that:
- the reaction of the product of formula (II) with the product of formula (III) is carried out in an organic solvent such as ethyl ether, tetrahydrofuran or dimethoxymethane;
- the salts of the products of formula (II) and ($I_B$) can be prepared by treatment of the corresponding product of formula (II) or ($I_B$) by a reagent capable of forming an anion, for example an alkali metal hydride or carbonate such as sodium hydride or potassium carbonate;
- the reaction of the product of formula ($I_B$) with the product of formula (IV) is carried out in an organic solvent such as tetrahydrofuran or dimethylformamide;
- when the eliminable group X of the product of formula (IV) represents an alkanoyloxy radical containing 1 to 6 carbon atoms, the resulting anhydride can serve as reagent and solvent;
- the esterification of the product of formula ($I_D$) is carried out according to standard methods, by treating, for example, the carboxylic acid of formula ($I_D$), or a reactive derivative of this acid, with an alcohol of formula (V);

  R'-OH (V)

  in which R' represents an alkyl radical containing 1 to 5 carbon atoms, preferably in a mineral acid such as hydrochloric acid and under reflux of the reaction medium;
- the alcohol of formula (V) can serve advantageously as the solvent;
- the nitration reaction is carried out according to standard methods, by treating, for example, the product of formula ($I_F$) by a system capable of producing "in situ" $NO_2^+$ ions, such as the mixture of sodium nitrate, lauthane nitrate in the presence of a mineral acid such as hydrochloric acid;
- this reaction is advantageously carried out in an organic solvent such as ethyl ether, at low temperature between 0° and 5° C, for example.

**10.** Medicaments, characterized in that they are constituted by new substituted 8-phenylthiotetrahydroquinolines as defined by formula (I) of claim 1, as well as their addition salts with pharmaceutically acceptable acids.

**11.** Medicaments, characterized in that they are constituted by new substituted 8-phenylthiotetrahydroquinolines as defined in any one of claims 2 or 3, as well as their addition salts with pharmaceutically acceptable acids.

**12.** Medicaments, characterized in that they are constituted by new substituted 8-phenylthiotetrahydroquinolines as defined in claim 4.

**13.** Pharmaceutical compositions, characterized in that they contain as active ingredient, at least one of the medicaments as defined in any one of claims 10 to 12.

Claims for the following Contracting States : AT, ES, GR

**1.** Preparation process for substituted 8-phenylthiotetrahydroquinolines corresponding to formula (I):

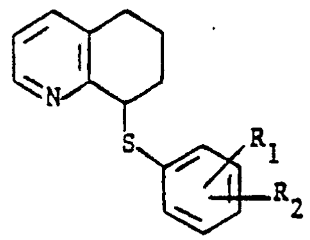

(I)

in which $R_1$ and $R_2$, identical or different, represent either a hydrogen atom, or a halogen atom, or an alkyl radical containing 1 to 6 carbon atoms optionally substituted by a hydroxyl radical, or an alkoxy radical containing 1 to 6 carbon atoms, or an alkanoyloxy radical containing 1 to 6 carbon atoms, or an alkoxycarbonyl radical containing 2 to 6 carbon atoms, or a hydroxy, nitro, amino, carboxy, cyano or aminosulphonyl radical, or when $R_1$ and $R_2$ are carried by the adjacent carbon atoms, form with these a phenyl radical, as well as their addition salts with acids, characterized in that a compound of formula (II):

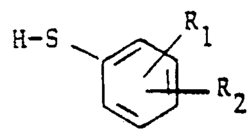

(II)

in the form of the salt, in which formula $R_1$ and $R_2$ have the meaning already indicated, is reacted with a compound of formula (III):

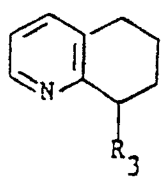

(III)

in which $R_3$ represents a halogen atom such as chlorine, bromine or iodine, in order to obtain a compound of formula (I) which, if desired, is salified.

2. Variant of the process according to claim 1, characterized in that the product of formula ($I_B$):

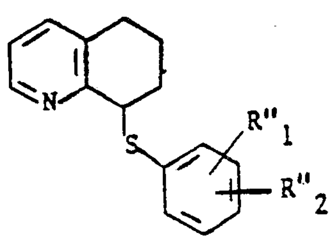

($I_B$)

in the form of the salt, in which formula $R''_1$ and $R''_2$ have the meaning indicated for $R_1$ and $R_2$, it being understood that at least one of $R''_1$ and $R''_2$ represents a hydroxy radical, is reacted with a product of formula (IV) :

R-X (IV)

in which R represents an alkyl or alkanoyl radical containing 1 to 6 carbon atoms and X represents an easily eliminable group such as a halogen atom or an alkanoyloxy radical in order to obtain a product of formula ($I_A$) :

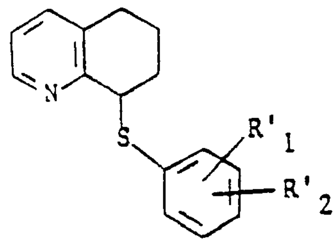

($I_A$)

in which $R'_1$ and $R'_2$ have the meaning indicated for $R_1$ and $R_2$, it being understood that at least one of $R'_1$ and $R'_2$ represents an alkoxy or alkanoyloxy radical containing 1 to 6 carbon atoms, which, if desired, is salified.

**3.** Variant of the process according to claim 1, characterized in that the product of formula ($I_D$):

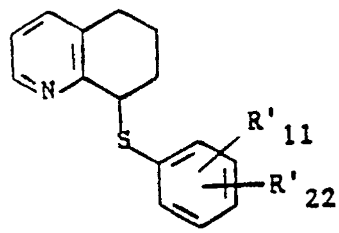

($I_D$)

in which $R'_{11}$ and $R'_{22}$ have the meaning already indicated for $R_1$ and $R_2$, it being understood that at least one of $R'_{11}$ and $R'_{22}$ represents a carboxy radical, is esterified in order to obtain a product of formula ($I_C$):

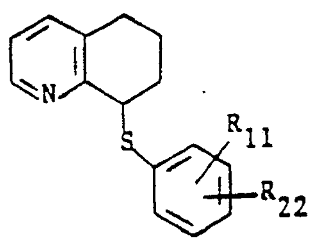

($I_C$)

in which $R_{11}$ and $R_{22}$ have the meaning already indicated for $R_1$ and $R_2$, it being understood that at least one of $R_1$ and $R_2$ represents an alkoxycarbonyl radical containing 2 to 6 carbon atoms, which, if desired is salified.

**4.** Variant of the process according to claim 1, characterized in that the nitration of the product of formula ($I_F$):

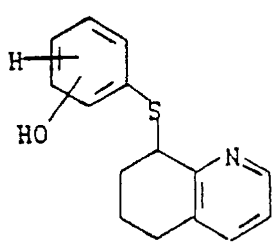

($I_F$)

is carried out in order to obtain a product of formula ($I_E$):

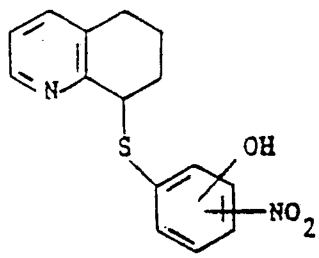

($I_E$)

which, if desired, is salified.

5. Preparation process or its variants according to one of claims 1 to 4, characterized in that:
   - the reaction of the product of formula (II) with the product of formula (III) is carried out in an organic solvent such as ethyl ether, tetrahydrofuran or dimethoxymethane;
   - the salts of the products of formula (II) and ($I_B$) can be prepared by treatment of the corresponding product of formula (II) or ($I_B$) by a reagent capable of forming an anion, for example an alkali metal hydride or carbonate such as sodium hydride or potassium carbonate;
   - the reaction of the product of formula ($I_B$) with the product of formula (IV) is carried out in an organic solvent such as tetrahydrofuran or dimethylformamide;
   - when the eliminable group X of the product of formula (IV) represents an alkanoyloxy radical containing 1 to 6 carbon atoms, the resulting anhydride can serve as reagent and solvent;
   - the esterification of the product of formula ($I_D$) is carried out according to standard methods, by treating, for example, the carboxylic acid of formula ($I_D$), or a reactive derivative of this acid, with an alcohol of formula (V);

     R'-OH (V)

     in which R' represents an alkyl radical containing 1 to 5 carbon atoms, preferably in a mineral acid such as hydrochloric acid and under reflux of the reaction medium;
   - the alcohol of formula (V) can serve advantageously as the solvent;
   - the nitration reaction is carried out according to standard methods, by treating, for example, the product of formula ($I_F$) by a system capable of producing "in situ" $NO_2^+$ ions, such as the mixture of sodium nitrate, lauthane nitrate in the presence of a mineral acid such as hydrochloric acid;
   - this reaction is advantageously carried out in an organic solvent such as ethyl ether, at low temperature between 0° and 5° C for example.

6. Preparation process according to claim 1 or 5, characterized in that at the start a product of formula (II) is used in which $R_1$ and $R_2$, identical of different, represent either a hydrogen atom, or a chlorine atom, or an alkyl radical containing 1 to 6 carbon atoms optionally substituted by a hydroxy radical, or an alkoxy radical containing 1 to 6 carbon atoms, or an alkanoyloxy radical containing 1 to 6 carbon atoms, or an alkoxy-carbonyl radical containing 2 to 6 carbon atoms, or a hydroxy radical, or a nitro radical, or when $R_1$ and $R_2$ are carried by the adjacent carbon atoms form with these a phenyl radical.

7. Preparation process according to claim 1 or 5, characterized in that at the start a product of formula (II) is used in which $R_1$ represents a hydrogen atom and $R_2$ represents a hydrogen or a chlorine atom or a methyl, n-butyl, methoxy, n-butoxy, 1-hydroxyhexyl, hydroxy, acetoxy or nitro radical or when $R_1$ and $R_2$ are carried by the two adjacent carbon atoms they form with these a phenyl radical.

8. Preparation process according to any one of claims 1 to 7, characterized in that the following are prepared:
   - 8-(1-naphthylthio)-5,6,7,8-tetrahydroquinoline,
   - 8-(4-butylphenylthio)-5,6,7,8-tetrahydroquinoline and its hydrochloride,
   - 8-(2-naphthylthio)-5,6,7,8-tetrahydroquinoline and its hydrochloride,
   - 8-(4-methylphenylthio)-5,6,7,8-tetrahydroquinoline and its hydrochloride.

9. Preparation process of pharmaceutical compositions, characterized in that at least one of the products

of formula (I) as obtained in claim 1 or at least one of its pharmaceutically acceptable salts is used as active ingredient in a form adapted for therapeutic use.

**10.** Preparation process of pharmaceutical compositions, characterized in that at least one of the products of formula (I) as obtained in any one of claims 2 to 7 or at least one of its pharmaceutically acceptable salts is used as active ingredient in a form adapted for therapeutic use.

**11.** Preparation process of pharmaceutical compositions, characterized in that at least one of the products of formula (I) as obtained in claim 8 or at least one of its pharmaceutically acceptable salts is used as active ingredient in a form adapted for therapeutic use.

## Ansprüche

**1.** Substituierte 8-Phenylthiotetrahydrochinoline entsprechend der Formel (I)

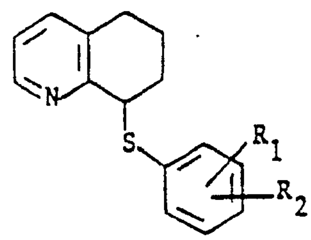

(I)

worin $R_1$ und $R_2$, die identisch oder verschieden sind, entweder ein Wasserstoffatom oder ein Halogenatom oder einen Alkylrest mit 1 bis 6 Kohlenstoffatomen, gegebenenfalls substituiert durch einen Hydroxylrest,oder einen Alkoxyrest mit 1 bis 6 Kohlenstoffatomen oder einen Alkanoyloxyrest mit 1 bis 6 Kohlenstoffatomen oder einen Alkoxycarbonylrest mit 2 bis 6 Kohlenstoffatomen oder einen Hydroxy-, Nitro-, Amino-, Carboxy-, Cyano- oder Aminosulfonylrest bedeuten oder, wenn $R_1$ und $R_2$ von benachbarten Kohlenstoffatomen getragen sind, mit diesen einen Phenylrest bilden, sowie ihre Additionssalze mit Säuren.

**2.** Substituierte 8-Phenylthiotetrahydrochinoline, wie in Anspruch 1 definiert, und ihre Additionssalze mit Säuren, dadurch gekennzeichnet, daß in dieser Formel (I) $R_1$ und $R_2$, die identisch oder verschieden sind, entweder ein Wasserstoffatom oder ein Chloratom oder einen Alkylrest mit 1 bis 6 Kohlenstoffatomen, gegebenenfalls substituiert durch einen Hydroxylrest, oder einen Alkoxyrest mit 1 bis 6 Kohlenstoffatomen oder einen Alkanoyloxyrest mit 1 bis 6 Kohlenstoffatomen oder einen Alkoxycarbonylrest mit 2 bis 6 Kohlenstoffatomen oder einen Hydroxyrest oder einen Nitrorest bedeuten oder wenn $R_1$ und $R_2$ von benachbarten Kohlenstoffatomen getragen sind, mit diesen einen Phenylrest bilden.

**3.** Substituierte 8-Phenylthiotetrahydrochinoline, wie sie in Anspruch 1 oder 2 definiert sind, sowie ihre Additionssalze mit Säuren, dadurch gekennzeichnet, daß in der Formel (I) $R_1$ ein Wasserstoffatom bedeutet und $R_2$ ein Wasserstoff- oder Chloratom wiedergibt oder einen Methyl-, n-Butyl-, Methoxy-, n-Butoxy-, 1-Hydroxyhexyl-, Hydroxy-, Acetoxy- oder Nitrorest bedeutet oder wenn $R_1$ und $R_2$ von 2 benachbarten Kohlenstoffatomen getragen sind, mit diesen einen Phenylrest bilden.

**4.** Derivate der Formel (I) gemäß Anspruch 1, insbesondere namentlich
8-(1-Naphthylthio)-5,6,7,8-tetrahydrochinolin,
8-(4-Butylphenylthio)-5,6,7,8-tetrahydrochinolin und sein Hydrochlorid,
8-(2-Naphthylthio)-5,6,7,8-tetrahydrochinolin und sein Hydrochlorid,
8-(4-Methylphenylthio)-5,6,7,8-tetrahydrochinolin und sein Hydrochlorid.

**5.** Verfahren zur Herstellung von neuen, substituierten 8-Phenylthiotetrahydrochinolinen, wie sie in Formel (I) von Anspruch 1 definiert sind, sowie ihrer Salze, dadurch gekennzeichnet, daß man eine Verbindung der Formel (II)

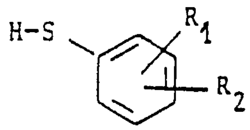

(II)

in Salzform, worin $R_1$ und $R_2$ die bereits angegebenen Bedeutungen haben, mit einer Verbindung der Formel (III)

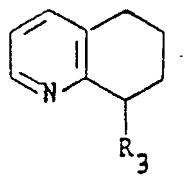

(III)

worin $R_3$ ein Halogenatom, wie Chlor, Brom oder Jod, bedeutet, zur Reaktion bringt, um die Verbindung der Formel (I) zu erhalten, die gewünschtenfalls in ein Salz übergeführt werden kann.

6. Variante des Herstellungsverfahrens gemäß Anspruch 5, dadurch gekennzeichnet, daß man das Produkt der Formel ($I_B$)

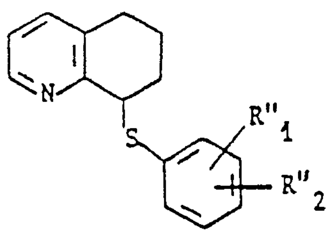

($I_B$)

in Salzform, worin $R''_1$ und $R''_2$ die für $R_1$ und $R_2$ angegebene Bedeutung haben, wobei mindestens eines von $R''_1$ und $R''_2$ einen Hydroxyrest bedeutet, mit einem Produkt der Formel (IV)

R - X (IV)

worin R einen Alkyl- oder Alkanoylrest mit 1 bis 6 Kohlenstoffatomen bedeutet und X eine leicht entfernbare Gruppe darstellt, wie ein Halogenatom oder ein Alkanoyloxyrest, zur Reaktion bringt, um ein Produkt der Formel ($I_A$)

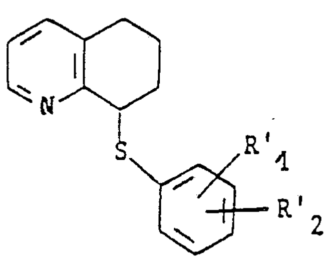

($I_A$)

zu erhalten, worin $R'_1$ und $R'_2$ die für $R_1$ und $R_2$ angegebene Bedeutung haben, wobei jedoch mindestens eines von $R'_1$ und $R'_2$ einen Alkoxy- oder Alkanoyloxyrest mit 1 bis 6 Kohlenstoffatomen bedeutet, welches man gegebenenfalls in ein Salz überführt.

7. Variante des Herstellungsverfahrens gemäß Anspruch 5, dadurch gekennzeichnet, daß man das Produkt der Formel ($I_D$)

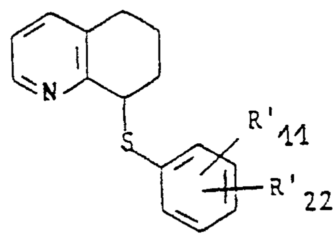

($I_D$)

verestert, worin $R'_{11}$ und $R'_{22}$ die für $R_1$ und $R_2$ angegebene Bedeutung haben, wobei jedoch mindestens eines von $R'_{11}$ und $R'_{22}$ einen Carboxyrest bedeutet, um ein Produkt der Formel ($I_C$)

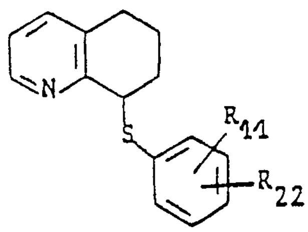

($I_C$)

zu erhalten, worin $R_{11}$ und $R_{22}$ die für $R_1$ und $R_2$ angegebene Bedeutung haben, wobei jedoch mindestens eines von $R_1$ und $R_2$ einen Alkoxycarbonylrest mit 2 bis 6 Kohlenstoffatomen bedeutet, das man gewünschtenfalls in ein Salz überführt.

**8.** Variante des Verfahrens gemäß Anspruch 5, dadurch gekennzeichnet, daß man die Nitrierung des Produkts der Formel ($I_F$)

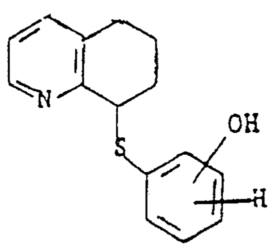

($I_F$)

durchführt, um ein Produkt der Formel ($I_E$)

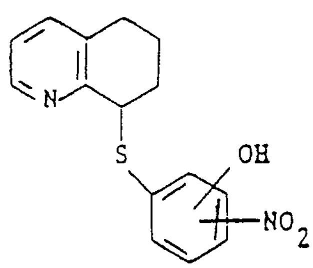

($I_E$)

zu erhalten, das man gewünschtenfalls in ein Salz überführt.

**9.** Herstellungsverfahren oder seine Varianten gemäß einem der Ansprüche 5 bis 8, dadurch gekennzeichnet, daß

- die Reaktion des Produkts der Formel (II) mit dem Produkt der Formel (III) in einem organischen Lösungsmittel, wie Ethylether, Tetrahydrofuran oder Dimethoxymethan, durchgeführt wird;
- die Salze der Produkte der Formel (II) und ($I_B$) hergestellt werden können durch Behandlung des entsprechenden Produkts der Formel (II) oder ($I_B$) mit einem Reaktanten, der ein Anion zu bilden vermag, beispielsweise ein Alkalimetallhydrid oder -carbonat, wie Natriumhydrid oder Kaliumcarbonat;

- die Reaktion des Produkts der Formel ($I_B$) mit dem Produkt der Formel (IV) in einem organischen Lösungsmittel, wie Tetrahydrofuran oder Dimethylformamid, durchgeführt wird;
- wenn die entfernbare Gruppe X des Produkts der Formel (IV) einen Alkanoyloxyrest mit 1 bis 6 Kohlenstoffatomen bedeutet, das entstandene Anhydrid als Reaktionsteilnehmer und Lösungsmittel dienen kann;
- die Veresterung des Produkts der Formel ($I_D$) nach üblichen Methoden durchgeführt wird, indem beispielsweise die Carbonsäure der Formel ($I_D$) oder ein reaktives Derivat dieser Säure mit einem Alkohol der Formel (V)

  R' - OH (V)

  worin R' einen Alkylrest mit 1 bis 5 Kohlenstoffatomen bedeutet, behandelt wird, vorzugsweise in einer Mineralsäure, wie Chlorwasserstoffsäure, und unter Rückfluß des Reaktionsmilieus;
- der Alkohol der Formel (V) vorteilhaft als Lösungsmittel dienen kann;
- die Nitrierungsreaktion nach üblichen Methoden durchgeführt wird, indem beispielsweise das Produkt der Formel ($I_F$) mit einem System behandelt wird, das in der Lage ist, "in situ" $NO_2^+$ -Ionen zu produzieren, wie das Gemisch Natriumnitrat, Lanthannitrat in Gegenwart einer Mineralsäure, wie Salzsäure;
- diese Reaktion vorteilhaft in einem organischen Lösungsmittel, wie Ethylether, bei niedriger Temperatur zwischen 0 und 5 °C beispielsweise durchgeführt wird.

**10.** Arzneimittel, dadurch gekennzeichnet, daß sie aus den neuen, substituierten 8-Phenylthiotetrahydrochinolinen, wie sie durch die Formel (I) von Anspruch 1 definiert sind, bestehen sowie ihren Additionssalzen mit pharmazeutisch annehmbaren Säuren.

**11.** Arzneimittel, dadurch gekennzeichnet, daß sie aus den neuen, substituierten 8-Phenylthiotetrahydrochinolinen bestehen, wie sie in einem der Ansprüche 2 oder 3 definiert sind, sowie ihren Additionssalzen mit pharmazeutisch annehmbaren Säuren.

**12.** Arzneimittel, dadurch gekennzeichnet, daß sie aus den neuen, substituierten 8-Phenylthiotetrahydrochinolinen, wie sie in Anspruch 4 definiert sind, bestehen.

**13.** Pharmazeutische Zusammensetzungen, dadurch gekennzeichnet, daß sie als aktives Prinzip mindestens eines der Arzneimittel, wie sie in einem der Ansprüche 10 bis 12 definiert sind, enthalten.

Patentansprüche für folgende Vertragsstaaten : AT, ES, GR

**1.** Verfahren zur Herstellung von substituierten 8-Phenylthiotetrahydrochinolinen der Formel (I)

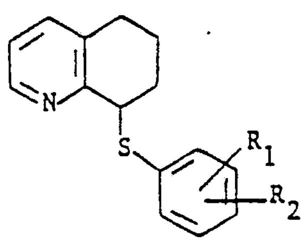

(I)

worin $R_1$ und $R_2$, die identisch oder verschieden sind, entweder ein Wasserstoffatom oder ein Halogenatom oder einen Alkylrest mit 1 bis 6 Kohlenstoffatomen, gegebenenfalls substituiert durch einen Hydroxylrest, oder einen Alkoxyrest mit 1 bis 6 Kohlenstoffatomen oder einen Alkanoyloxyrest mit 1 bis 6 Kohlenstoffatomen oder einen Alkoxycarbonylrest mit 2 bis 6 Kohlenstoffatomen oder einen Hydroxy-, Nitro-, Amino-, Carboxy-, Cyano- oder Aminosulfonylrest bedeuten, oder wenn $R_1$ und $R_2$ von benachbarten Kohlenstoffatomen getragen sind, mit diesen einen Phenylrest bilden, sowie von ihren Additionssalzen mit Säuren, dadurch gekennzeichnet, daß man eine Verbindung der Formel (II)

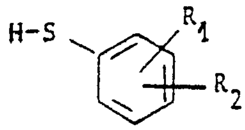

(II)

in Salzform, worin $R_1$ und $R_2$ die bereits angegebenen Bedeutungen haben, mit einer Verbindung der Formel (III)

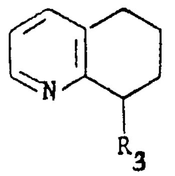

(III)

worin $R_3$ ein Halogenatom, wie Chlor, Brom oder Jod, bedeutet, zur Reaktion bringt, um die Verbindung der Formel (I) zu bilden, die gewünschtenfalls in ein Salz übergeführt werden kann.

**2.** Variante des Verfahrens gemäß Anspruch 1, dadurch gekennzeichnet, daß man das Produkt der Formel ($I_B$)

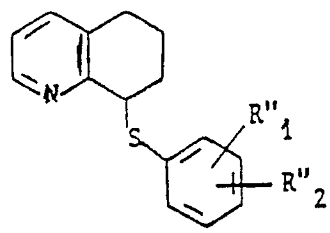

($I_B$)

in Salzform, worin $R''_1$ und $R''_2$ die für $R_1$ und $R_2$ angegebene Bedeutung haben, wobei mindestens eines von $R''_1$ und $R''_2$ einen Hydroxyrest bedeutet, mit einem Produkt der Formel (IV)

R - X (IV)

worin R einen Alkyl- oder Alkanoylrest mit 1 bis 6 Kohlenstoffatomen bedeutet und X eine leicht entfernbare Gruppe darstellt, wie ein Halogenatom oder ein Alkanoyloxyrest, zur Reaktion bringt, um ein Produkt der Formel ($I_A$)

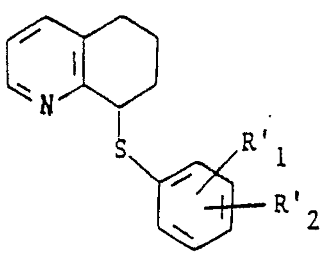

($I_A$)

zu erhalten, worin $R'_1$ und $R'_2$ die für $R_1$ und $R_2$ angegebene Bedeutung haben, wobei jedoch mindestens eines von $R'_1$ und $R'_2$ einen Alkoxy- oder Alkanoyloxyrest mit 1 bis 6 Kohlenstoffatomen bedeutet, welches man gegebenenfalls in ein Salz überführt.

**3.** Variante des Verfahrens gemäß Anspruch 1, dadurch gekennzeichnet, daß man das Produkt der Formel ($I_D$)

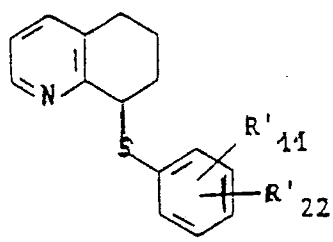

$(I_D)$

verestert, worin $R'_{11}$ und $R'_{22}$ die für $R_1$ und $R_2$ angegebene Bedeutung haben, wobei jedoch mindestens eines von $R'_{11}$ und $R'_{22}$ einen Carboxyrest bedeutet, um ein Produkt der Formel $(I_C)$

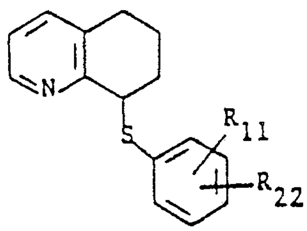

$(I_C)$

zu erhalten, worin $R_{11}$ und $R_{22}$ die für $R_1$ und $R_2$ angegebene Bedeutung haben, wobei jedoch mindestens eines von $R_1$ und $R_2$ einen Alkoxycarbonylrest mit 2 bis 6 Kohlenstoffatomen bedeutet, das man gewünschtenfalls in ein Salz überführt.

**4.** Variante des Verfahrens gemäß Anspruch 1, dadurch gekennzeichnet, daß man die Nitrierung des Produkts der Formel $(I_F)$

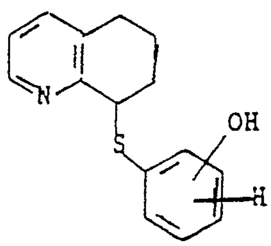

$(I_F)$

durchführt, um ein Produkt der Formel $(I_E)$

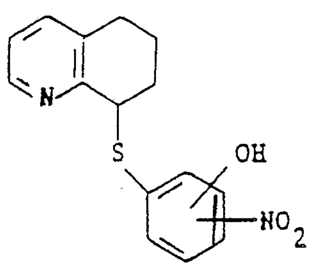

$(I_E)$

zu erhalten, das man gewünschtenfalls in ein Salz überführt.

**5.** Verfahren zur Herstellung oder Abwandlung gemäß einem der Ansprüche 1 bis 4, dadurch gekennzeichnet, daß

- die Reaktion des Produkts der Formel (II) mit dem Produkt der Formel (III) in einem organischen Lösungsmittel, wie Ethylether, Tetrahydrofuran oder Dimethoxymethan, durchgeführt wird;
- die Salze der Produkte der Formel (II) und $(I_B)$ hergestellt werden können durch Behandlung des entsprechenden Produkts der Formel (II) oder $(I_B)$ mit einem Reaktanten, der ein Anion zu bilden vermag, beispielsweise ein Alkalimetallhydrid oder -carbonat, wie Natriumhydrid oder Kaliumcarbonat;
- die Reaktion des Produkts der Formel ($I_B$) mit dem Produkt der Formel (IV) in einem organischen Lösungsmittel, wie Tetrahydrofuran oder Dimethylformamid, durchgeführt wird;
- wenn die entfernbare Gruppe X des Produkts der Formel (IV) einen Alkanoyloxyrest mit 1 bis 6 Kohlenstoffatomen bedeutet, das entstandene Anhydrid als Reaktionsteilnehmer und Lösungsmittel dienen kann;
- die Veresterung des Produkts der Formel ($I_D$) nach üblichen Methoden durchgeführt wird, indem beispielsweise die Carbonsäure der Formel ($I_D$) oder ein reaktives Derivat dieser Säure mit einem Alkohol der Formel (V)

  R' - OH (V)

  worin R' einen Alkylrest mit 1 bis 5 Kohlenstoffatomen bedeutet, behandelt wird, vorzugsweise in einer Mineral-Säure, wie Chlorwasserstoffsäure, und unter Rückfluß des Reaktionsmilieus;
- der Alkohol der Formel (V) vorteilhaft als Lösungsmittel dienen kann;
- die Nitrierungsreaktion nach üblichen Methoden durchgeführt wird, indem beispielsweise das Produkt der Formel ($I_F$) mit einem System behandelt wird, das in der Lage ist, "in situ" $NO_2^+$ -Ionen zu produzieren, wie das Gemisch Natriumnitrat, Lanthannitrat in Gegenwart einer Mineralsäure, wie Salzsäure;
- diese Reaktion vorteilhaft in einem organischen Lösungsmittel, wie Ethylether, bei niedriger Temperatur zwischen 0 und 5 °C beispielsweise durchgeführt wird.

**6.** Verfahren zur Herstellung gemäß Anspruch 1 oder 5, dadurch gekennzeichnet, daß man zu Beginn ein Produkt der Formel (II) verwendet, worin $R_1$ und $R_2$, die identisch oder verschieden sind, entweder ein Wasserstoffatom oder ein Chloratom oder einen Alkylrest mit 1 bis 6 Kohlenstoffatomen, gegebenenfalls substituiert durch einen Hydroxyrest,oder einen Alkoxyrest mit 1 bis 6 Kohlenstoffatomen oder einen Alkanoyloxyrest mit 1 bis 6 Kohlenstoffatomen oder einen Alkoxycarbonylrest mit 2 bis 6 Kohlenstoffatomen oder einen Hydroxyrest oder einen Nitrorest bedeuten oder wenn $R_1$ und $R_2$ von benachbarten Kohlenstoffatomen getragen sind, mit diesen einen Phenylrest bilden.

**7.** Herstellungsverfahren gemäß Anspruch 1 oder 5, dadurch gekennzeichnet, daß man zu Beginn ein Produkt der Formel (II) verwendet, worin $R_1$ ein Wasserstoffatom bedeutet und $R_2$ ein Wasserstoff- oder Chloratom oder èinen Methyl-, n-Butyl-, Methoxy-, n-Butoxy-, 1-Hydroxy-hexyl-, Hydroxy-, Acetoxy- oder Nitrorest bedeutet oder wenn $R_1$ und $R_2$ von 2 benachbarten Kohlenstoffatomen getragen sind, mit diesen einen Phenylrest bilden.

**8.** Herstellungsverfahren gemäß einem der Ansprüche 1 bis 7, dadurch gekennzeichnet, daß man herstellt:
8-(1-Naphthylthio)-5,6,7,8-tetrahydrochinolin,
8-(4-Butylphenylthio)-5,6,7,8-tetrahydrochinolin und sein Hydrochlorid,
8-(2-Naphthylthio)-5,6,7,8-tetrahydrochinolin und sein Hydrochlorid,
8-(4-Methylphenylthio)-5,6,7,8-tetrahydrochinolin und sein Hydrochlorid.

**9.** Verfahren zur Herstellung pharmazeutischer Zusammensetzungen, dadurch gekennzeichnet, daß man als aktives Prinzip wenigstens eines der Produkte der Formel (I), erhalten gemäß Anspruch 1, oder mindestens eines seiner pharmazeutisch annehmbaren Salze in eine Form bringt, die für die therapeutische Verwendung angepaßt ist.

**10.** Verfahren zur Herstellung pharmazeutischer Zusammensetzungen, dadurch gekennzeichnet, daß man als aktives Prinzip wenigstens eines der Produkte der Formel (I), wie in einem der Ansprüche 2 bis 7 erhalten, oder mindestens eines seiner pharmazeutisch annehmbaren Salze in eine Form bringt, die zur therapeutischen Anwendung angepaßt ist.

**11.** Verfahren zur Herstellung pharmazeutischer Zusammensetzungen, dadurch gekennzeichnet, daß man als aktives Prinzip wenigstens eines der Produkt der Formel (I), wie gemäß Anspruch 8 erhalten, oder wenigstens eines seiner pharmazeutisch annehmbaren Salze in eine Form bringt, die zur therapeutischen Verwendung angepaßt ist.